# EUROPEAN PATENT APPLICATION

(11) **EP 4 494 589 A1**
(43) Date of publication of application: **22.01.2025**
(21) Application number: 24189332.0
(22) Date of filing: 18.07.2024
(51) Int. Cl.: A61B 18/14, A61B 18/00

(54) **ELECTRODE ATTACHMENTS FOR BASKET CATHETERS**

(30) Priority: 19.07.2023 US 202363514504 P; 18.06.2024 US 202418747119
(71) Applicant: Biosense Webster (Israel) Ltd., 2066717 Yokneam (IL)
(72) Inventor: SUAREZ, Paul, Irvine, 92618 (US); ROSEBERRY, Jacob, Irvine, 92618 (US); PATEL, Amar, Irvine, 92618 (US); NGUYEN, Thanh, Irvine, 92618 (US)
(74) Representative: Carpmaels & Ransford LLP

(57) **Abstract**

The disclosed technology includes electrode attachments for a medical probe, including an electrode body configured to deliver electrical energy to biological tissues. Elongated member(s) of the medical probe can include one or more apertures, and the electrode(s) can include one or more electrode apertures to align with the apertures of the elongated member. A pin is placed into the aligned apertures to secure the electrode to the elongated member.

## Description

### CROSS REFERENCE TO RELATED APPLICATIONS

This application claims the benefit of priority under 35 U.S.C. § 119 to prior filed U.S. Provisional Patent Application No. 63/514,504, filed July 19, 2023 (Attorney Docket No.: BIO6842USPSP1 - 253757.000368), the entire contents of which is hereby incorporated by reference as if set forth in full herein.

### FIELD

The present invention relates generally to medical devices, and in particular medical probes with electrodes, and further relates to, but not exclusively, medical probes suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference and attached in the Appendix hereto.

Regions of cardiac tissue can be mapped by a catheter to identify the abnormal electrical signals. The same or different medical probe can be used to perform ablation. Some example probes include a number of spines with electrodes positioned thereon. The electrodes are generally attached to the spines and secured in place by soldering, welding, or using an adhesive. Due to the small size of the spines and the electrodes, however, soldering, welding, or adhering the electrodes to the spines can be a difficult task, increasing the manufacturing time and cost and the chances that the electrode fails due to an improper bond, misalignment, or strain on the spine. What is needed, therefore, are systems and methods of attaching an electrode to a spine of a basket assembly without the need for soldering, welding, or using adhesive.

### SUMMARY

There is provided, in accordance with an embodiment of the present invention, a spine assembly. The assembly can include an elongated member extending along a longitudinal axis member comprising a first aperture along a length of the member. The assembly can include a first electrode slidable along the length of the member. The first electrode comprising a first electrode aperture. The assembly can include a first pin disposed within the first aperture and the first electrode aperture when the first aperture and the first electrode aperture are aligned along the length of the member.

The first pin can include a non-conductive material. The first electrode aperture can have a non-conductive internal surface. A pin head of the first pin can have a beveled profile.

The first pin can include a rivet having a bottom that is compressible to form a second head opposite a first head. The first head and the second head of the rivet can be positioned at opposing sides of the first electrode.

The assembly can include a second electrode slidable along the length of the member. The second electrode can include a second electrode aperture. The assembly can include a second pin. The member can include a second aperture along the length of the member. The second pin can be disposed within the second aperture and the second electrode aperture when the second aperture and the second electrode aperture are aligned along the length of the member.

The assembly can include a second pin. The member can include a second aperture. The first electrode can include a second electrode aperture, and the second pin can be disposed within the second aperture and the second electrode aperture when the second aperture and the second electrode aperture are aligned along the length of the member. The assembly can include a first tab and a second tab. The first tab can include the first electrode aperture, and the second tab can include the second electrode aperture. The first tab and the second tab can be positioned at opposite ends of the first electrode in a direction along the length of the member. The second electrode aperture can have a non-conductive internal surface.

The disclosed technology can include an expandable basket assembly. The assembly can include at least one spine extending along a longitudinal axis and configured to bow radially outward from the longitudinal axis. The spine can include a first spine aperture and a second spine aperture disposed along a length of the spine. The assembly can include a first electrode slidable along the length of the spine. The first electrode can include a first electrode aperture and a second electrode aperture. The assembly can include a first pin disposed within the first spine aperture and the first electrode aperture when the first spine aperture and the first electrode aperture are aligned along the length of the spine. The assembly can include a second pin disposed within the second spine aperture and the second electrode aperture when the second spine aperture and the second electrode aperture are aligned along the length of the spine.

The first pin and the second pin can include a non-conductive material. The first electrode aperture and the second electrode aperture can have non-conductive internal surfaces. A first pin head of the first pin can have a beveled profile, and a second pin head of the second pin can have a beveled profile. The first pin and the second pin can include rivets that are compressible to form a beveled end.

The assembly can include a first tab and a second tab. The first tab can include the first electrode aperture, and the second tab can include the second electrode aperture. The first tab and the second tab can be positioned at opposite ends of the first electrode in a direction along the length of the spine. In some examples, the first tab and the second tab can be connected to and extending from the first electrode. The first electrode aperture and the second electrode aperture can have non-conductive internal surfaces.

The disclosed technology can include an expandable basket assembly. The assembly can include at least one spine extending along a longitudinal axis. The spine can include a first spine aperture along a length of the spine. The assembly can include a first electrode slidable along the length of the spine. The first electrode can include a first electrode aperture. The assembly can include a first rivet disposed within the first spine aperture and the first electrode aperture when the first spine aperture and the first electrode aperture are aligned along the length of the spine.

The first rivet can include a non-conductive material. The first electrode aperture can have a non-conductive internal surface. A rivet head of the first rivet can have a beveled profile. The rivet can include a multi-piece component having a rivet top connectable to a rivet bottom. The rivet top and the rivet bottom can be positioned at opposing sides of the first electrode when connected.

The assembly can include a second electrode slidable along the length of the spine, the second electrode comprising a second electrode aperture. The assembly can include a second rivet. The spine can include a second spine aperture along the length of the spine. The second rivet can be disposed within the second spine aperture and the second electrode aperture when the second spine aperture and the second electrode aperture are aligned along the length of the spine.

The assembly can include a second rivet. The spine can include a second spine aperture. The first electrode can include a second electrode aperture. The second rivet can be disposed within the second spine aperture and the second electrode aperture when the second spine aperture and the second electrode aperture are aligned along the length of the spine.

The assembly can include a first tab and a second tab. The first tab can include the first electrode aperture, and the second tab can include the second electrode aperture. The first tab and the second tab can be positioned at opposite ends of the first electrode in a direction along the length of the spine. The second electrode aperture can have a non-conductive internal surface.

The disclosed technology can include an expandable basket assembly. The assembly can include at least one spine extending along a longitudinal axis. The spine can include a first spine attachment along a length of the spine. The first spine attachment can be an aperture, or a position along a length of a slot along the length of the spine. The assembly can include a first electrode slidable along the length of the spine. The first electrode can include an extension insertable into the first spine attachment and compressible to form a compressed base.

The disclosed technology can further include a method of attaching an electrode to an elongated member. The method can include placing a first electrode at an end of the elongated member. The method can include sliding the first electrode along a length of the member until a first electrode aperture of the first electrode aligns with a first aperture of the member. The method can include inserting a first pin into the first electrode aperture and the first aperture to affix the first electrode to the member. Placing the first electrode at the end of the member can include placing the end of the member into a slot of the first electrode. The member being slidable through the slot.

The method can include inserting a second pin into a second electrode aperture and a second aperture to affix the first electrode to the member. The first electrode aperture can be disposed within a first tab, and the second electrode aperture can be disposed within a second tab. The first tab and the second tab can extend from a main body of the first electrode.

The first pin can include a rivet having a first rivet head. The method can further include deforming a bottom of the first pin to secure the first pin within the first electrode aperture and the first aperture. The first pin can include a rivet having a first rivet head. The method can further include connecting a rivet bottom to the first pin to secure the first pin within the first electrode aperture and the first aperture.

Sliding the first electrode along a length of the member until a first electrode aperture of the first electrode aligns with a first aperture of the member can include sliding the first electrode past a second aperture. The method can further include placing a second electrode at the end of the member. The method can further include sliding the second electrode along the length of the member until a second electrode aperture of the second electrode aligns with the second aperture. The method can further include inserting a second pin into the first electrode aperture and the second aperture to affix the second electrode to the member.

The disclosed technology can further include a method of attaching an electrode to a spine of a basket catheter. The method can include sliding a first electrode along a length of the spine until a first electrode aperture of the first electrode aligns with a first spine aperture of the spine, and a second electrode aperture of the first electrode aligns with a second spine aperture of the spine. The method can include inserting a first pin into the first electrode aperture and the first spine aperture to affix the first electrode to the spine. The method can include inserting a second pin into the second electrode aperture and the second spine aperture.

The method can include placing the end of the spine into a spine slot of the first electrode, the spine being slidable through the spine slot. The first electrode aperture can be disposed within a first tab, and the second electrode aperture can be disposed within a second tab. The first tab and the second tab can extend from a main body of the first electrode.

The first pin and the second pin are rivets can have a rivet head. The method can further include deforming bottoms of the first pin and the second pin to secure the pins within the first electrode aperture and the first spine aperture.

The first pin can include a rivet having a first rivet head. The second pin can include a rivet having a second rivet head. The method further includes connecting a first rivet bottom to the first pin to secure the first pin within the first electrode aperture and the first spine aperture. The method further includes connecting a second rivet bottom to the second pin to secure the second pin within the second electrode aperture and the second spine aperture.

Sliding the first electrode along a length of the spine until a first electrode aperture of the first electrode aligns with a first spine aperture of the spine can include sliding the first electrode past a third spine aperture and a fourth spine aperture. The method can further include sliding a second electrode along the length of the spine until a third electrode aperture of the second electrode aligns with a third spine aperture of the spine, and a fourth electrode aperture of the second electrode aligns with a fourth spine aperture of the spine. The method can further include inserting a third pin into the third electrode aperture and the third spine aperture to affix the second electrode to the spine. The method can further include inserting a fourth pin into the fourth electrode aperture and the fourth spine aperture.

The disclosed technology can further include a method of securing an electrode to an elongated member. The method can include sliding a first tab along a length of a spine until a first electrode aperture of the first tab aligns with a first spine aperture of the spine. The method can include inserting a first pin into the first electrode aperture and the first spine aperture to affix the first tab to the spine. The method can include sliding a first electrode along the length of the spine until the first electrode contacts the first tab. The method can include sliding a second tab along the length of the spine until a second electrode aperture of the second tab aligns with a second spine aperture of the spine. The method can include inserting a second pin into the second electrode aperture and the second spine aperture to affix the second tab to the spine.

The disclosed technology can further include a method of attaching an electrode to a spine of a basket catheter. The method can include sliding a first electrode along a length of the spine until a first electrode aperture of the first electrode aligns with a first spine aperture of the spine. The method can include inserting a first rivet into the first electrode aperture and the first spine aperture. The method can include riveting the first rivet to affix the first electrode to the spine.

The method can include placing the end of the spine into a spine slot of the first electrode. The spine can be slidable through the spine slot. Riveting the first rivet can include connecting a rivet bottom to the first rivet to affix the first electrode to the spine. Sliding the first electrode along a length of the spine until a first electrode aperture of the first electrode aligns with a first spine aperture of the spine can include sliding the first electrode past a second spine aperture. The method can further include sliding a second electrode along a length of the spine until a second electrode aperture of the second electrode aligns with the second spine aperture. The method can further include inserting a second rivet into the second electrode aperture and the second spine aperture. The method can further include riveting the second rivet to affix the second electrode to the spine.

The disclosed technology can further include a method of securing an electrode to an elongated member. The method can include sliding a first electrode along a length of a spine until it reaches a spine attachment on the spine. The method can include compressing an extension of the first electrode to form a compressed base at an end of the extension, thereby securing the first electrode to the spine at a location of the spine attachment.

### BRIEF DESCRIPTION OF THE DRAWINGS

FIG. 1 is a schematic pictorial illustration of a medical system including a medical probe with a distal end that includes a medical probe with electrodes, in accordance with an embodiment of the present invention;
FIG. 2A is a schematic pictorial illustration showing a perspective view of a medical probe with electrodes in an expanded form, in accordance with an embodiment of the present invention;
FIG. 2B is a schematic pictorial illustration showing a side view of a medical probe in a collapsed form, in accordance with the disclosed technology;
FIG. 3A is a schematic pictorial illustration showing a top view of a spine and an electrode positioned thereon, in accordance with the disclosed technology;
FIG. 3B is a schematic pictorial illustration showing a top view of a spine and an electrode positioned at a final seated position on the spine, in accordance with the disclosed technology;
FIG. 3C is a schematic pictorial illustration showing a top view of an electrode attached to a spine with a pin, in accordance with the disclosed technology;
FIG. 3D is a schematic pictorial illustration showing a side view of the electrode attached to the spine as shown in FIG. 3C, in accordance with the disclosed technology;
FIG. 3E is a schematic pictorial illustration showing an end view of the electrode of FIGs. 3A-3D, in accordance with the disclosed technology;
FIG. 3F is a schematic pictorial illustration showing an example pin, in accordance with the disclosed technology;
FIG. 4A is a schematic pictorial illustration showing a top view of a spine and an electrode positioned thereon, in accordance with the disclosed technology;
FIG. 4B is a schematic pictorial illustration showing a top view of a spine and an electrode positioned at a final seated position on the spine, in accordance with the disclosed technology;
FIG. 4C is a schematic pictorial illustration showing a top view of an electrode attached to a spine with two pins, in accordance with the disclosed technology;
FIG. 4D is a schematic pictorial illustration showing a side view of the electrode attached to the spine as shown in FIG. 4C, in accordance with the disclosed technology;
FIG. 4E is a schematic pictorial illustration showing an end view of the electrode of FIGs. 4A-4D, in accordance with the disclosed technology;
FIG. 4F is a schematic pictorial illustration showing an example pin, in accordance with the disclosed technology;
FIG. 5A is a schematic pictorial illustration showing a top view of a spine and an electrode positioned thereon, in accordance with the disclosed technology;
FIG. 5B is a schematic pictorial illustration showing a top view of a spine and an electrode positioned at a final seated position on the spine, in accordance with the disclosed technology;
FIG. 5C is a schematic pictorial illustration showing a top view of an electrode attached to a spine with a pin comprising a rivet, in accordance with the disclosed technology;
FIG. 5D is a schematic pictorial illustration showing a side view of the electrode attached to the spine as shown in FIG. 5C, in accordance with the disclosed technology;
FIG. 5E is a schematic pictorial illustration showing an end view of the electrode of FIGs. 5A-5D, in accordance with the disclosed technology;
FIG. 5F is a schematic pictorial illustration showing an example deformable rivet, in accordance with the disclosed technology;
FIG. 5G is a schematic pictorial illustration showing an example multi-piece rivet, in accordance with the disclosed technology;
FIG. 6A is a schematic pictorial illustration showing an electrode with an extension and a compressible base, in accordance with the disclosed technology;
FIG. 6B is a schematic pictorial illustration showing an electrode with an extension attached to a spine, in accordance with the disclosed technology;
FIG. 6C is a schematic pictorial illustration showing an electrode with an extension that has a rectangular cross section, in accordance with the disclosed technology;
FIG. 6D is a schematic pictorial illustration showing an electrode with an extension that has a rectangular cross section slid along a slot in a spine, in accordance with the disclosed technology;
FIG. 7 is a flowchart illustrating a method of attaching an electrode to an elongated member, in accordance with an embodiment of the present invention;
FIG. 8 is a flowchart illustrating a method of attaching an electrode to a spine of a basket catheter, in accordance with an embodiment of the present invention;
FIG. 9 is a flowchart illustrating a method of securing an electrode to an elongated member, in accordance with an embodiment of the present invention;
FIG. 10 is a flowchart illustrating a method of attaching an electrode to a spine of a basket catheter, in accordance with an embodiment of the present invention; and
FIG. 11 is a flowchart illustrating a method of securing an electrode to an elongated member, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, method or uses and devices for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference and attached in the Appendix hereto.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference and attached in the Appendix hereto.

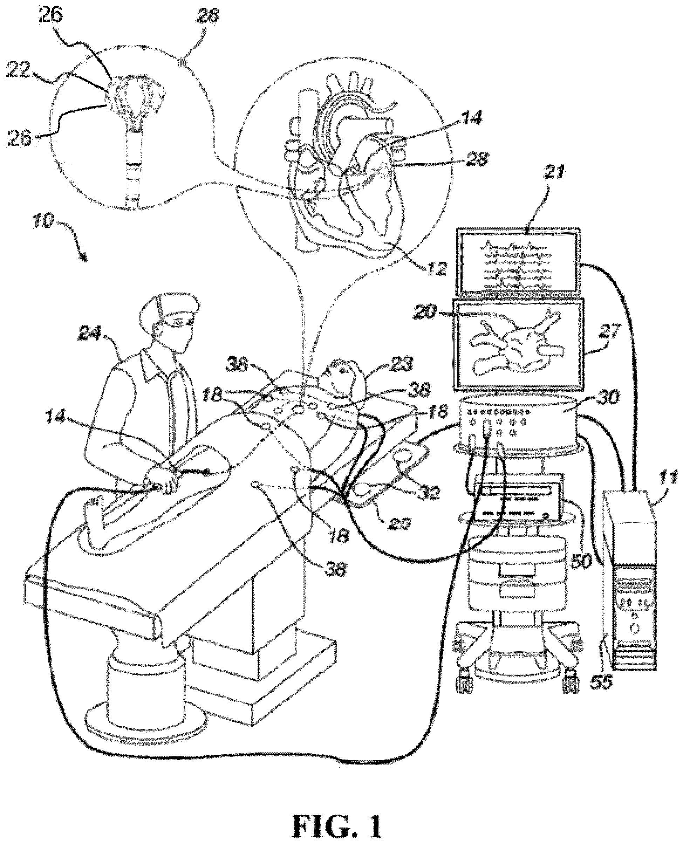

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip of catheter 14 (i.e., a basket assembly 28 in this case) into contact with the heart wall for sensing a target site in heart 12. A catheter 14 with a distal basket assembly can be referred to as a basket catheter. For ablation, physician 24 would similarly bring a distal end of an ablation catheter to a target site for ablating.

Catheter 14 is an exemplary catheter that includes one and preferably multiple electrodes 26 optionally distributed over a plurality of spines 22 at basket assembly 28 and configured to sense the IEGM signals. Catheter 14 may additionally include a position sensor embedded in or near basket assembly 28 for tracking position and orientation of basket assembly 28. Optionally and preferably, position sensor is a magnetic based position sensor including three magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of basket assembly 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference and attached in the Appendix hereto.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference and attached in the Appendix hereto.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTOTM 3 System, available from Biosense Webster, Inc., 31 Technology Drive, Suite 200, Irvine, CA 92618 USA.

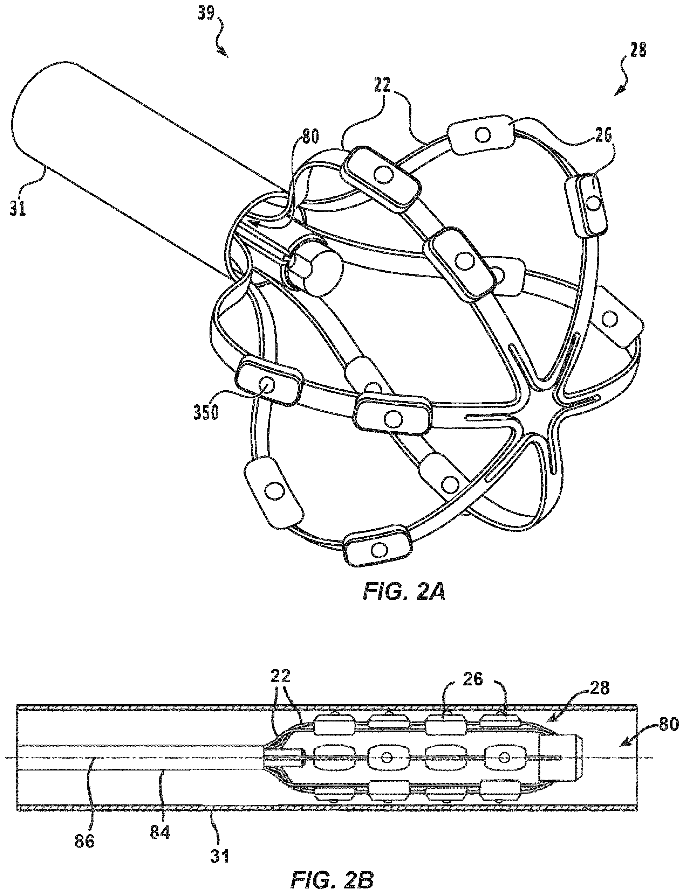

FIG. 2A is a schematic pictorial illustration showing a perspective view of a medical probe 39 with electrodes 26 in an expanded form, such as by being advanced out of a tubular shaft lumen 80 at a distal end of a tube 31, in accordance with an embodiment of the present invention. FIG. 2B shows a medical probe 39 with electrodes 26 in a collapsed form within tube 31. In the expanded form (FIG. 2A), one or more spines 22 bow radially outwardly and in the collapsed form (FIG. 2B) the spines 22 are arranged generally along a longitudinal axis 86 of tubular shaft 84.

As shown in FIG. 2A, the medical probe 39 includes a plurality of flexible spines 22 that are formed at the end of a tubular shaft 84 and can be connected at their ends. During a medical procedure, medical professional 34 can deploy medical probe 39 by extending tubular shaft 84 from tube 31 causing the medical probe 39 to exit the tube 31 and transition to the expanded form. Spines 22 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

As will be appreciated by one skilled in the art with the benefit of this disclosure, the basket assembly 28 shown in FIGs. 2A-2B having spines 22 formed from a single sheet of planar material and converging at a central intersection is offered merely for illustrative purposes and the disclosed technology can be applicable to other configurations of basket assemblies 28. For example, the disclosed technology can be applicable to basket assemblies 28 formed from a single spine 22 or multiple spines 22 with each spine 22 being attached at both ends. In other examples, the basket assembly 28 can include a central hub connecting the multiple spines 22 together at a distal end of the basket assembly 28. In yet other examples, the basket assembly 28 can include a single spine 22 configured to form a spiral, multiple spines 22 configured to form a spiral, multiple spines 22 configured to form a tripod or multiple tripods, or any other shape of basket assembly 28. Thus, although FIGs. 2A-2B illustrate a specific configuration of basket assembly 28, the disclosed technology should not be construed as so limited. As well, the basket assembly 28 can be formed by laser cutting a cylindrical hollow stock material whereby the laser is mounted for rotation about the longitudinal axis (and translation thereto) of the cylindrical stock while cutting. It will be understood that catheter devices that include a basket assembly 28 (as shown herein) cam be referred to as basket catheters.

The spines 22 can be formed from a single sheet of planar material to form a generally star shape. In other words, the spines 22 can be formed from the single sheet of planar material such that the spines 22 converge toward a central intersection. The intersection can be a solid piece of material or include one or more apertures.

As will be appreciated, the spine 22 can be electrically isolated from the electrode 26 to prevent arcing from the electrode 26 to the spine 22. For example, insulative jackets can be positioned between the spine(s) 22 and the electrode(s) 26, but one of skill in the art will appreciate that other insulative coverings are contemplated. For example, an insulative coating can be applied to the spine 22, the electrodes 26, or both. The insulative jackets can be made from a biocompatible, electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets may also include one or more additives or fillers, such as, for example, polytetrafluoroethylene (PTFE), boron nitride, silicon nitride, silicon carbide, aluminum oxide, aluminum nitride, zinc oxide, and the like.

In embodiments described herein, electrodes 26 can be configured to deliver ablation energy (RF and/or IRE) to tissue in heart 12. In addition to using electrodes 26 to deliver ablation energy, the electrodes can also be used to determine the location of medical probe 39 and/or to measure a physiological property such as local surface electrical potentials at respective locations on tissue in heart 12. The electrodes 26 can be biased such that a greater portion of the electrode 26 faces outwardly from the medical probe 39 such that the electrodes 26 deliver a greater amount of electrical energy outwardly away from the medical probe 39 (i.e., toward the heart 12 tissue) than inwardly toward the medical probe 39.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

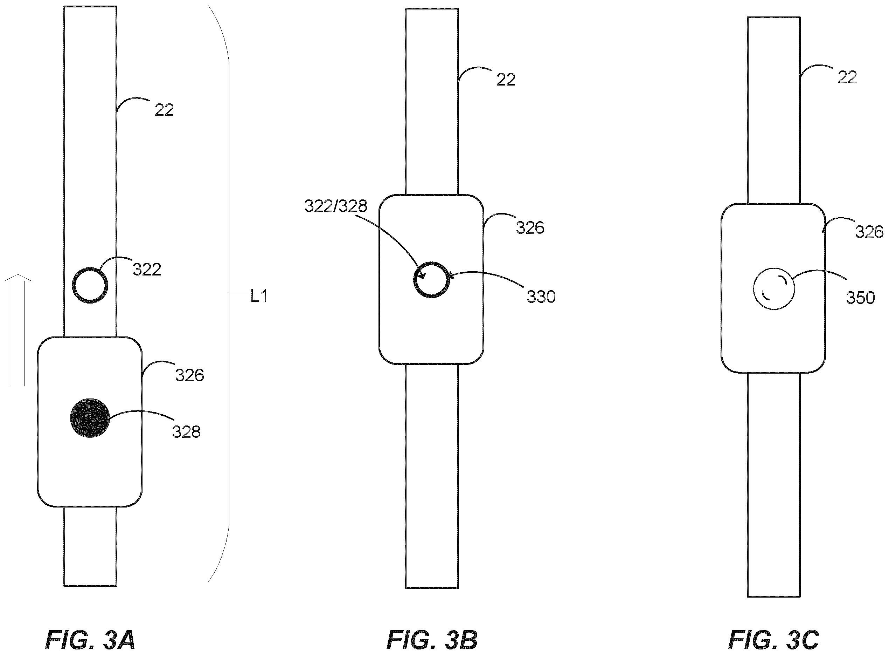

FIGs. 3A-3F are schematic pictorial illustrations showing an electrode 326 and its engagement with a medical probe 39. FIGs. 3A-3C show the process of assembly and attachment of an electrode 326 onto a member of the medical probe 39, for example onto a spine 22 of the medical probe 39. The electrode 326 shown in FIGs. 3A-3F can be similar to the electrode 26 described above. In FIG. 3A, the electrode 326 is not fully attached but is partially slid along a spine 22. The spine 22 can include an aperture 322 positioned along a length L1 of the spine 22 that corresponds to an intended position of the electrode 326. The aperture 322 can align with an electrode aperture 328 on the electrode 326 when the electrode 326 is seated into its assembled position. FIG. 3B shows the electrode 326 slid into its seated position such that the aperture 322 aligns with the electrode aperture 328. At this point, a pin 350 can be inserted into the aligned aperture 322/electrode aperture 328 so as to connect the electrode 326 at its final position on the spine 22 (see FIG. 3C).

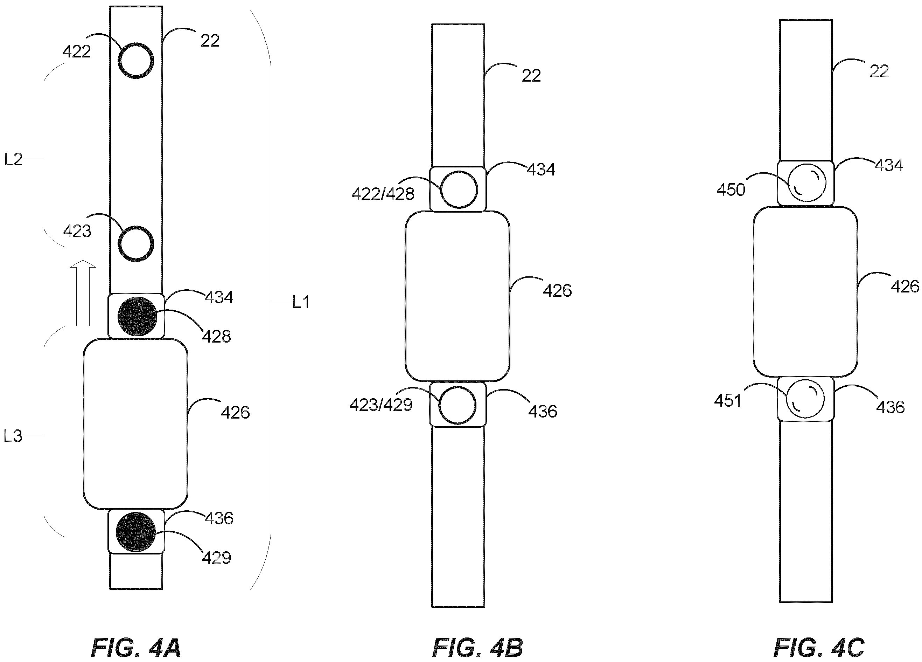

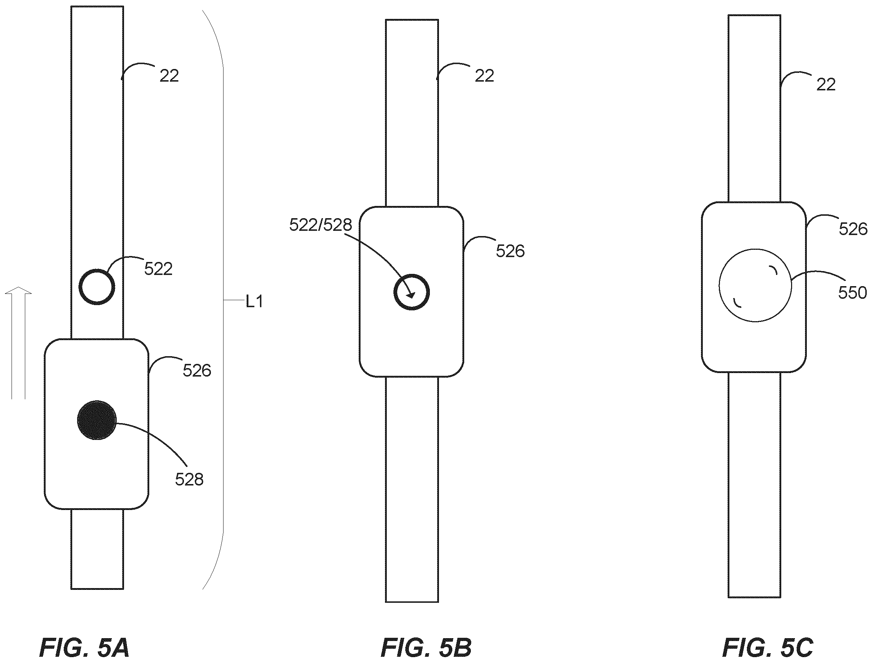

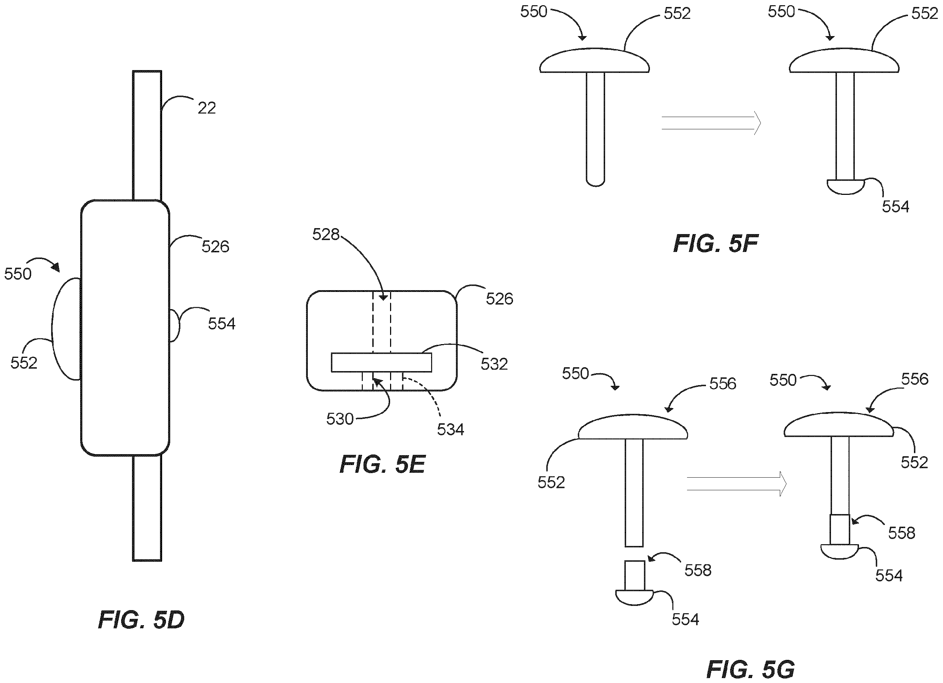

Assembly in the manner shown and described in FIGs. 3A-3D (and as described below with respect to FIGs. 4A-4D and 5A-5D) enables the medical probe 39 to be assembled without the use of certain locking mechanisms that may make it difficult to seat more than one electrode along the spine 22. For example, and referring to FIG. 2A for illustration, certain implementations of basket catheters include more than one electrode along the length L1 of one spine. To seat the distalmost electrode, the electrode must be slid past a proximal electrode placement location. A system that includes attachment features (e.g., tabs and the like) formed directly onto the spine to engage with the electrodes makes it difficult to advance the distalmost electrode past the proximal electrode placement location. The present design enables easier placement of the distalmost electrode since the locking mechanism on the spine 22 itself is an aperture, e.g., aperture 322, and the distalmost electrode can be easily slid past the aperture. Accordingly, it will be appreciated that each spine 22 can have one electrode, two electrodes, and so forth, and each of the electrodes can be attached to the spine 22 according to any of the embodiments described in this disclosure.

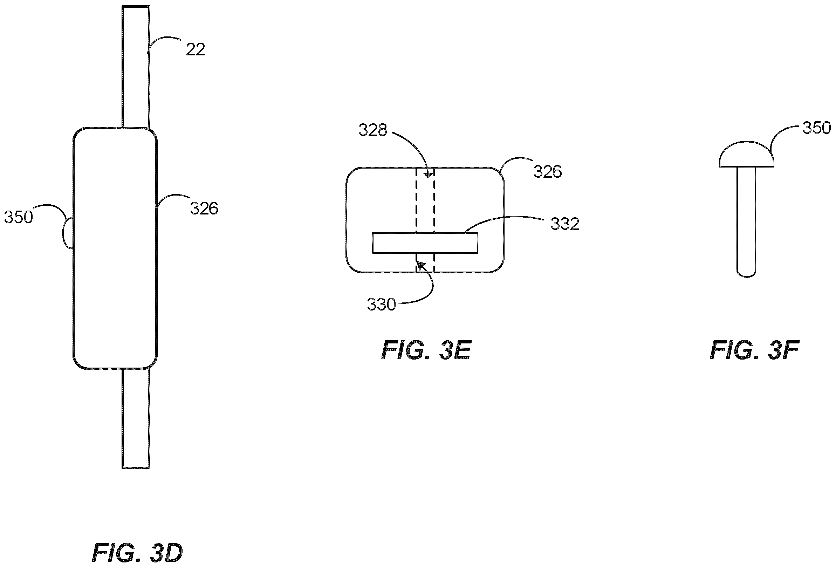

Referring to FIG. 3E, which shows a disassembled electrode 326, the electrode 326 can have a rectangular cross-section, as shown, or another cross-sectional geometry such as an elliptical (e.g., circular) cross-section. The electrode aperture 328 can have a non-conductive internal surface 330. For example, the inside of the aperture that accepts the pin 350 can be coated with a biocompatible, electrically insulative material such as Pebax copolymers, PET, urethanes, polyimide, parylene, silicone, PEEK, PGA, PLGA, PCL, PHBV, poly-L-lactide, polydioxanone, polycarbonates, and/or the like. Insulating the junction between the pin 350 and the electrode aperture 328 can protect the pin 350 from becoming the point of electrical conduction, and as such the entire upper surface of the electrode 326 continues to be the ablation surface. The electrode 326 can have a spine slot 332 disposed therethrough that can be sized and shaped to accept the spine 22. The electrode 326 can therefore slide down the length L1 of the spine 22 via engagement of the spine 22 within the spine slot 332. In some examples, the internal surface of the spine slot 332 can have a coating to provide additional friction between the electrode 326 and the spine 22, such as silicone and the like. In an alternative embodiment, the perimeter of the spine aperture 322 can be coated in a non-conductive material, and the pin 350 and the electrode aperture 328 can remain conductive. A goal is to keep the electrode 326 and the spine 22 electrically isolated from each other, but the surfaces that touch (i.e., the pin 350, the electrode aperture, and spine aperture 322) can be interchanged such that there is an insulative connection between the surfaces.

Referring to FIG. 3F, the pin 350 can be manufactured from a non-conductive material. For example, the pin 350 can be made from a biocompatible, electrically insulative material such as Pebax copolymers, PET, urethanes, polyimide, parylene, silicone, PEEK, PGA, PLGA, PCL, PHBV, poly-L-lactide, polydioxanone, polycarbonates, and/or the like. Alternatively, the pin 350 can be manufactured from a metallic material or another potentially conductive material. In these examples, the pin 350 can be coated in said non-conductive material. The non-conductivity can, again, protect the pin 350 from becoming the point of electrical conduction, and as such the entire upper surface of the electrode 326 continues to be the ablation surface. The pin 350 can include features to assist in securing the pin 350 inside of the electrode aperture 328. For example, the pin 350 can be a ball lock pin, a threaded pin, a spring pin, and the like. In some examples, the pin 350 can be a rivet, as will be described herein with respect to FIGs. 5A-5G.

In some examples, a head of the pin 350 can have a beveled profile so as to provide an atraumatic profile (see FIG. 3D). In some examples, the head of the pin 350 can extend above the surface of the electrode 326 as shown in FIG. 3D. In other examples the head of the pin 350 may be recessed into the body of the electrode. For example, the electrode aperture 328 can also include a countersunk portion to house, and lower, the head of the pin 350.

FIGs. 4A-4F are schematic pictorial illustrations showing an electrode 426 and its engagement with a medical probe 39. FIGs. 4A-4C show the process of assembly and attachment of an electrode 426 onto a member of the medical probe 39, for example onto a spine 22 of the medical probe 39. The electrode 426 shown in FIGs. 4A-4F can be similar to electrode 26 and electrode 326 described above. The electrode 426 in FIGs. 4A-4F provides two points of attachment to a spine 22. Referring to FIG. 4A, the electrode 426 is not fully attached but is partially slid down a length L1 of a spine 22. The spine 22 can include a first spine aperture 422 positioned along the length L1 of the spine 22, and can include a second spine aperture 423 positioned along the length L1 of the spine 22. The electrode 426 can include a first electrode aperture 428 and a second electrode aperture 429. The distance L2 between the first spine aperture 422 and the second spine aperture 423 can be the same as the distance L3 between the first electrode aperture 428 and the second electrode aperture 429. FIG. 4B shows the electrode 426 slid into its seated position such that the first electrode aperture 428 aligns with the first spine aperture 422, and the second electrode aperture 429 aligns with the second spine aperture 423. At this point, a first pin 450 can be inserted into the aligned first spine aperture 422/first electrode aperture 428, and a second pin 451 can be inserted into the second spine aperture 423/second electrode aperture 429 (see FIG. 4C).

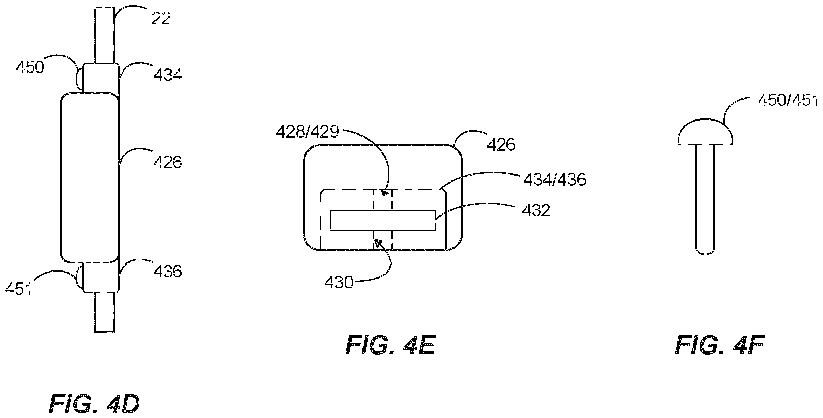

Referring again to FIG. 4A, in some examples the electrode 426 can include tabs extending from the electrode body so that the pins 450/451 do not interfere with the ablating surface of the electrode 426. The side view in FIG. 4D also shows an example of this layout, wherein the electrode 426 is positioned between a first tab 434 that includes the first electrode aperture 428, and a second tab 436 that includes the second electrode aperture 429. The first tab 434 and the second tab 436 can have a shorter profile than the main body of the electrode 426 such that, once the pins 450/451 are inserted into their respective apertures, the heads of the pins 450/451 do not extend above the top surface of the electrode 426. In some examples, the first tab 434 and the second tab 436 can be attached to or integrated with the electrode 426. For example, the first tab 434 and the second tab 436 can extend as wings from the ends of the electrode 426. In this example, the electrode 426 can be slid along with the tabs to the respective apertures and then secured. In another example, the first tab 434 and the second tab 436 can be separate components that secure the electrode 426 between the two. For example, a distalmost of the two tabs (e.g., tab 434 in FIG. 4A) can be slid to its respective spine aperture 422 and secured with a pin 450. The electrode 426 can be slid to contact the distal tab 434, and then the proximal tab (tab 436 in FIG. 4A) can be slid to its respective spine aperture 423 and secured with a pin 451.

Referring to FIG. 4E, which shows a disassembled electrode 426, the electrode 426 can have a rectangular cross-section, as shown, or another cross-sectional geometry such as an elliptical (e.g., circular) cross-section. In some examples, in some examples, the electrode apertures 428/429 can have a non-conductive internal surface 430. For example, the inside of the apertures that accepts the pins 450/451 can be coated with a biocompatible, electrically insulative material such as Pebax copolymers, PET, urethanes, polyimide, parylene, silicone, PEEK, PGA, PLGA, PCL, PHBV, poly-L-lactide, polydioxanone, polycarbonates, and/or the like. Insulating the junction between the pins 450/451 and the electrode apertures 428/429 can protect the pins 450/451 from becoming a point of electrical conduction. The electrode 426 can have a spine slot 432 disposed therethrough that can be sized and shaped to accept the spine 22. The electrode 426 can therefore slide down the length L1 of the spine 22 via engagement of the spine 22 with the spine slot 432. In some examples, the internal surface of the spine slot 432 can have a coating to provide additional friction between the electrode 426 and the spine 22, such as silicone and the like.

Referring to FIG. 4F, the pins 450/451 can be manufactured from a non-conductive material. For example, the pins 450/451 can be made from a biocompatible, electrically insulative material such as Pebax copolymers, PET, urethanes, polyimide, parylene, silicone, PEEK, PGA, PLGA, PCL, PHBV, poly-L-lactide, polydioxanone, polycarbonates, and/or the like. Alternatively, the pins 450/451 can be manufactured from a metallic material or another potentially conductive material. In these examples, the pins 450/451 can be coated in said non-conductive material. The non-conductivity can, again, protect the pins 450/451 from becoming the point of electrical conduction, and as such the surface of the electrode 426 continues to be the ablation surface. The pins 450/451 can include features to assist in securing the pins 450/451 inside of the electrode apertures 428/429. For example, the pins 450/451 can be ball lock pins, threaded pins, spring pins, and the like. In some examples, the pins 450/451 can be a rivets, as will be described with respect to FIGs. 5A-5G.

In some examples, a head of the pins 450/451 can have a beveled profile so as to provide an atraumatic profile (see FIG. 4D). In some examples, the head of the pins 450/451 can extend from the surfaces of their respective tabs 432/434 as shown in FIG. 4D. In other examples the head of the pins 450/451 may be recessed into the body of their respective tabs 432/434. For example, the electrode apertures 428/429 can also include a countersunk portion to house, and lower, the heads of the pins 450/451.

FIGs. 5A-5G are schematic pictorial illustrations showing an electrode 526 and its engagement with a medical probe 39. FIGs. 5A-5C show the process of assembly and attachment of an electrode 526 onto a member of the medical probe 39, for example onto a spine 22 of the medical probe 39. The electrode 526 shown in FIGs. 5A-5G can be similar to electrode 26, electrode 326, and electrode 426 described above. In FIG. 5A, the electrode 526 is not fully attached but is partially slid along a spine 22. The spine 22 can include an aperture 522 positioned along a length L1 of the spine 22 that corresponds to an intended position of the electrode 526. The aperture 522 can align with an electrode aperture 528 on the electrode 526 when the electrode 526 is seated into its assembled position. FIG. 5B shows the electrode 526 slid into its seated position such that the aperture 522 aligns with the electrode aperture 528. A pin, in this case a rivet 550, can be inserted into the aligned aperture 522/electrode aperture 528 so as to connect the electrode 526 at its final position on the spine 22 (see FIG. 5C).

Referring to FIG. 5E, which shows a disassembled electrode 526, the electrode 526 can have a rectangular cross-section, as shown, or another cross-sectional geometry such as an elliptical (e.g., circular) cross-section. In some examples, in some examples, the electrode aperture 528 can have a non-conductive internal surface 530. For example, the inside of the aperture that accepts the rivet 550 can be coated with a biocompatible, electrically insulative material such as Pebax copolymers, PET, urethanes, polyimide, parylene, silicone, PEEK, PGA, PLGA, PCL, PHBV, poly-L-lactide, polydioxanone, polycarbonates, and/or the like. Insulating the junction between the rivet 550 and the electrode aperture 528 can protect the rivet 550 from becoming the point of electrical conduction, and as such the entire upper surface of the electrode 526 continues to be the ablation surface. The electrode 526 can have a spine slot 532 disposed therethrough that can be sized and shaped to accept the spine 22. The electrode 526 can therefore slide down the length L1 of the spine 22 via engagement of the spine 22 with the spine slot 532. In some examples, the internal surface of the spine slot 532 can have a coating to provide additional friction between the electrode 526 and the spine 22, such as silicone and the like.

Referring to FIGs. 5F and 5G, the rivet 550 can be manufactured from or include a non-conductive material. For example, the rivet 550 can be made from a biocompatible, electrically insulative material such as Pebax copolymers, PET, urethanes, polyimide, parylene, silicone, PEEK, PGA, PLGA, PCL, PHBV, poly-L-lactide, polydioxanone, polycarbonates, and/or the like. Alternatively, the rivet 550 can be manufactured from a metallic material or another potentially conductive material. In these examples, the rivet 550 can be coated in said non-conductive material. The non-conductivity can, again, protect the rivet 550 from becoming the point of electrical conduction, and as such the entire upper surface of the electrode 526 continues to be the ablation surface.

Referring to FIG. 5F, the rivet 550 can have a first rivet head 552 and a bottom that is compressible to form a second head 554 opposite a first rivet head 552. To assemble this type of deformable rivet 550, the rivet 550 can be inserted into the aligned aperture 522/electrode aperture 528 and then compressed such that the bottom of the rivet 550 is deformed to form the second head 554. Once the second head 554 is formed, the electrode 526 is affixed to the spine 22. The first rivet head 552 and/or second rivet head 554 can have an atraumatic profile, as shown in FIG. 5F.

Referring to FIG. 5G, the rivet 550 can be a multi-piece component comprising a rivet top 556 connectable to a rivet bottom 558. The rivet top 556 can have a first rivet head 552 and the rivet bottom 558 can have a second rivet head 554. The rivet top 556 can engage with the rivet bottom 558 to form a fixed rivet 550 to connect the electrode 526 to the spine 22. For example, the rivet bottom 558, on the end that is opposite its head 554, can have an opening larger than the bottom end of the rivet top 556 so that the rivet top 556 can be inserted into the rivet bottom 558. To assemble, the rivet top 556 can be inserted into the aligned aperture 522/electrode aperture 528, and then the rivet bottom 558 can be slid over and engaged with the bottom portion the rivet top 556 to secure the electrode 526 to the spine 22. The first rivet head 552 and the second rivet head 554 can have an atraumatic profile, as shown in FIG. 5G. In some examples, to account for the larger rivet bottom 558, the electrode aperture 528 in the electrode 526 can have an oversized portion 534 (see FIG. 5E) at one end to account for the larger diameter of the rivet bottom 558.

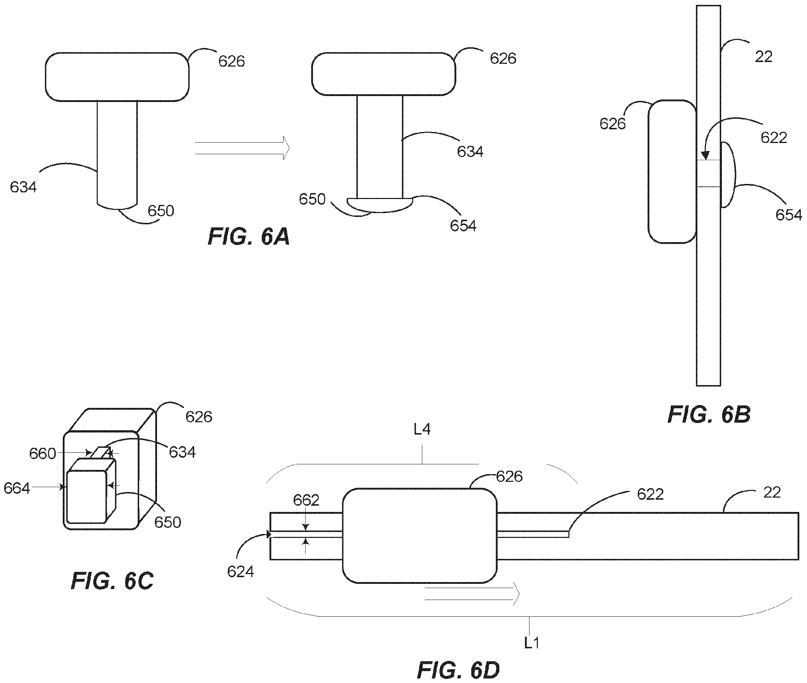

FIGs. 6A-6D are schematic pictorial illustrations showing an electrode 626 having an extension 634 with a compressible base, in accordance with the disclosed technology. For Other examples herein described an aperture within the electrode to accept a pin or a rivet. In this example shown in FIGs. 6A-6D, the electrode 626 can act as its own pin and rivet, as the extension 634 can be inserted into a spine attachment 622 of the spine 22 and then compressed to secure the electrode 626. The spine attachment 622 can be an aperture, such that an end 650 of the extension 634 can be inserted into the aperture, and then the end 650 of the extension 634 can be compressed to form a compressed base 654, in a manner similar to the second head 554 of the rivet described above. FIG. 6A shows an electrode 626 with an extension 634 being compressed. FIG. 6B shows the electrode 626 secured to a spine 622 by the end of the extension 634 being compressed into a compressed base 654 onto the spine 22.

In some examples, the spine attachment 622 can be a slot 624 extending a length L4 along the length L1 of the spine 22. The extension 634 of the electrode 626 can have a rectangular cross section with a width 660 that matches a width 662 of the slot 624. The extension 634 can be inserted into an end of the slot 624 and then slid to the desired location along the length L1 of the spine, and then the extension 634 can be compressed to secure the electrode 626 to the spine 22 at that location. In some examples, the end 650 of the extension 634 can have a larger footprint, or a larger width 664, than the remainder of the extension 634. As such, the extension 634 can be constrained within the slot 624 as it slides to its location along the spine 22.

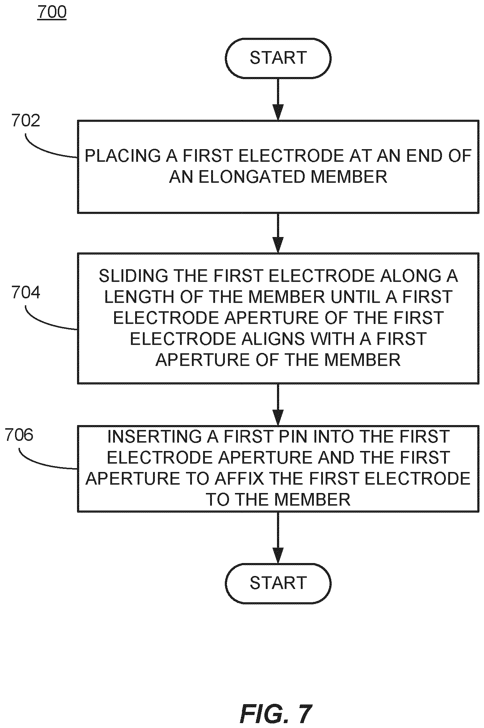

FIG. 7 is a flowchart illustrating a method 700 of attaching an electrode (e.g., electrode 26, 326, 426, and/or 526) to an elongated member, in accordance with an embodiment of the present invention. The method 700 can include placing 702 a first electrode at an end of the elongated member. The elongated member can be, for example, any of the spines 22 described herein. In some examples placing the first electrode at the end of the member can include placing the end of the member into a slot (e.g., spine slot 332) of the first electrode. The member can be slidable through the slot.

The method 700 can include sliding 704 the first electrode along a length of the member until a first electrode aperture of the first electrode aligns with a first aperture of the member. The method 700 can include inserting 706 a first pin (e.g., pin 350, pins 450/451, and/or rivet 550) into the first electrode aperture and the first aperture to affix the first electrode to the member.

The method 700 can end after the inserting step 706. In other examples, additional steps can be performed according to the examples described herein. For example, in some examples, the method 700 can include inserting a second pin into a second electrode aperture and a second aperture to affix the first electrode to the member via another pin.

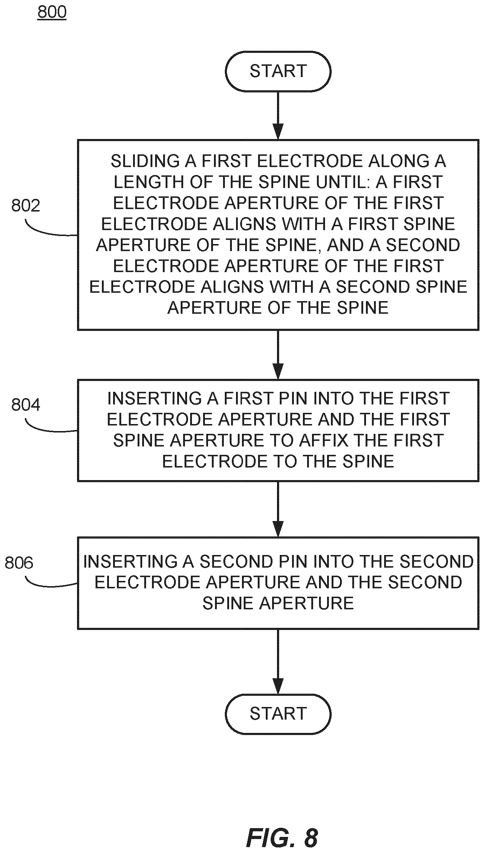

FIG. 8 is a flowchart illustrating a method 800 of attaching an electrode (e.g., electrode 26, 326, 426, and/or 526) to a spine (e.g., spine 22) of a basket catheter, in accordance with an embodiment of the present invention. The method 800 can include sliding 802 a first electrode along a length (e.g., L1) of the spine until (i) a first electrode aperture (e.g., 428) of the first electrode aligns with a first spine aperture (e.g., 422) of the spine, and (ii) a second electrode aperture (e.g., 429) of the first electrode aligns with a second spine aperture (e.g., 423) of the spine. In some examples, the electrode 428 can include tabs that include the electrode apertures (e.g., first tab 434 and second tab 436). In this case, sliding 802 the first electrode along a length of the spine can include sliding the electrode until (i) the first electrode aperture on the first tab 434 aligns with the first spine aperture, and the second electrode aperture on the second tab 436 aligns with the first spine aperture.

The method 800 can include inserting 804 a first pin (e.g., pin 350, pin 450, and/or rivet 550) into the first electrode aperture and the first spine aperture to affix the first electrode to the spine. The method 800 can include inserting 806 a second pin (e.g., pin 350, pin 451, and/or rivet 550) into the second electrode aperture and the second spine aperture.

The method 800 can end after the inserting step 806. In other examples, additional steps can be performed according to the examples described herein. For example, in some examples, the method 800 can include sliding a second electrode along the length of the spine until (iii) a third electrode aperture of the second electrode aligns with a third spine aperture of the spine and (iv) a fourth electrode aperture of the second electrode aligns with a fourth spine aperture of the spine. The method 800 can further include inserting a third pin into the third electrode aperture and the third spine aperture to affix the second electrode to the spine (e.g., step 804 can be repeated for the second electrode). The method 800 can further include inserting a fourth pin into the fourth electrode aperture and the fourth spine aperture to affix the second electrode to the spine (e.g., step 806 can be repeated for the second electrode).

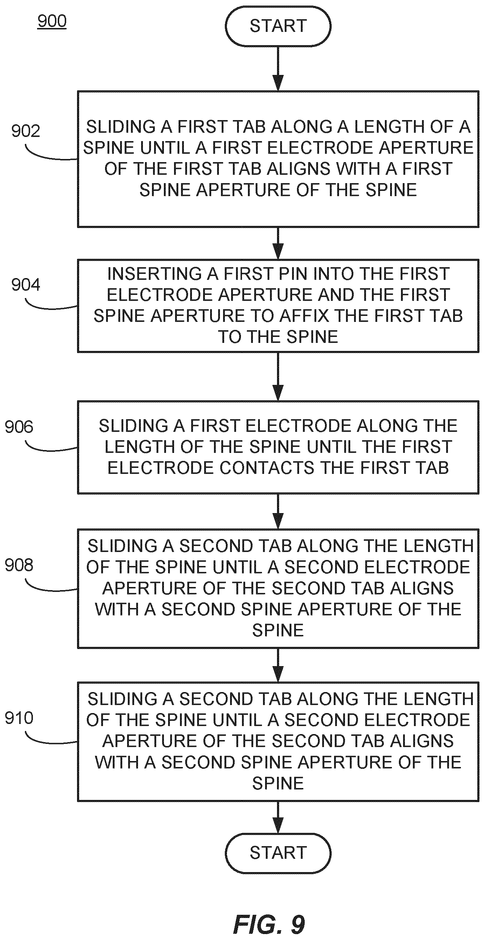

FIG. 9 is a flowchart illustrating a method 900 of securing an electrode (e.g., electrode 26, 326, 426, and/or 526) to an elongated member, in accordance with an embodiment of the present invention. This method 900 can be performed to secure an electrode using tabs (e.g., first tab 434 and second tab 436) that are separate from the electrode. The method 900 can include sliding 902 a first tab along a length of a spine until a first electrode aperture of the first tab aligns with a first spine aperture of the spine. The method 900 can include inserting 904 a first pin into the first electrode aperture and the first spine aperture to affix the first tab to the spine. The method 900 can include sliding 906 a first electrode along the length of the spine until the first electrode contacts the first tab. The method 900 can include sliding 908 a second tab along the length of the spine until a second electrode aperture of the second tab aligns with a second spine aperture of the spine. The method 900 can include inserting 910 a second pin into the second electrode aperture and the second spine aperture to affix the second tab to the spine.

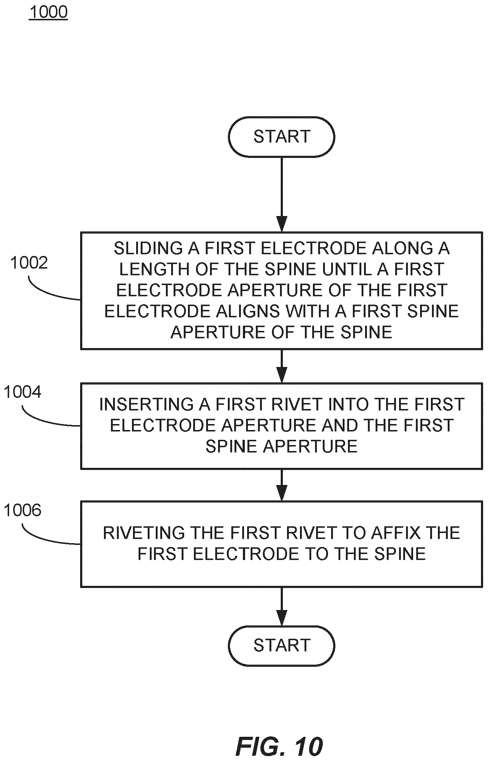

FIG. 10 is a flowchart illustrating a method 1000 of attaching an electrode (e.g., electrode 26, 326, 426, and/or 526) to a spine (e.g., spine 22) of a basket catheter, in accordance with an embodiment of the present invention. The method 1000 can include sliding 1002 a first electrode along a length of the spine until a first electrode aperture of the first electrode aligns with a first spine aperture of the spine. The method 1000 can include inserting 1004 a first rivet (e.g., rivet 550) into the first electrode aperture and the first spine aperture. The method 1000 can include riveting 1006 the first rivet to affix the first electrode to the spine. The method 1000 can end after the riveting step 1006. In other examples, additional steps can be performed according to the examples described herein.

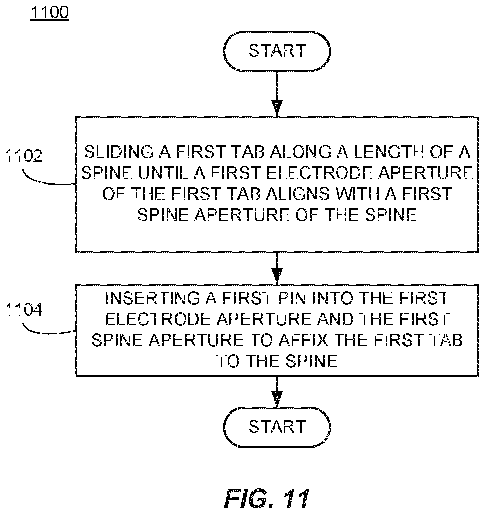

FIG. 11 is a flowchart illustrating a method 1100 of securing an electrode (e.g., electrode 26, 326, 426, 526, and/or 626) to an elongated member, in accordance with an embodiment of the present invention. The method 1100 can include sliding 1102 a first electrode along a length of a spine until it reaches a spine attachment on the spine. In some examples, the spine attachment can be a single aperture along the length of the spine. In other examples, the spine attachment can be slot, or track, along the length of the spine, and the spine attachment can be a position along the length of the slot, including at the end or any position along the length of the slot. The method 1100 can include compressing 1104 an extension of the first electrode to form a compressed base at an end of the extension, thereby securing the first electrode to the spine at a location of the spine attachment.

The disclosed technology described herein can be further understood according to the following clauses:
Clause 1. A spine assembly comprising: an elongated member extending along a longitudinal axis member comprising a first aperture along a length of the member; a first electrode slidable along the length of the member, the first electrode comprising a first electrode aperture; and a first pin disposed within the first aperture and the first electrode aperture when the first aperture and the first electrode aperture are aligned along the length of the member.
Clause 2. The assembly of Clause 1, the first pin comprises a non-conductive material.
Clause 3. The assembly of Clause 1 or 2, the first electrode aperture comprises a non-conductive internal surface.
Clause 4. The assembly of any of Clauses 1-3, a pin head of the first pin comprising a beveled profile.
Clause 5. The assembly of any of Clauses 1-4, the first pin comprises a rivet comprising a bottom that is compressible to form a second head opposite a first head.
Clause 6. The assembly of Clause 5, the first head and the second head of the rivet being positioned at opposing sides of the first electrode.
Clause 7. The assembly of any of Clauses 1-6 further comprising: a second electrode slidable along the length of the member, the second electrode comprising a second electrode aperture; and a second pin, the member comprising a second aperture along the length of the member, and the second pin is disposed within the second aperture and the second electrode aperture when the second aperture and the second electrode aperture are aligned along the length of the member.
Clause 8. The assembly of any of Clauses 1-6 further comprising a second pin, the member comprising a second aperture, the first electrode comprising a second electrode aperture, and the second pin being disposed within the second aperture and the second electrode aperture when the second aperture and the second electrode aperture are aligned along the length of the member.
Clause 9. The assembly of Clause 8 further comprising a first tab and a second tab, the first tab comprising the first electrode aperture, and the second tab comprising the second electrode aperture.
Clause 10. The assembly of Clause 9, the first tab and the second tab positioned at opposite ends of the first electrode in a direction along the length of the member.
Clause 11. The assembly of any of Clauses 8-10, the second electrode aperture comprises a non-conductive internal surface.
Clause 12. The assembly of any of Clauses 8-11, the first tab and the second tab being attached to and extending from the first electrode.
Clause 13. An expandable basket assembly comprising: at least one spine extending along a longitudinal axis and configured to bow radially outward from the longitudinal axis, the spine comprising a first spine aperture and a second spine aperture disposed along a length of the spine; a first electrode slidable along the length of the spine, the first electrode comprising a first electrode aperture and a second electrode aperture; a first pin disposed within the first spine aperture and the first electrode aperture when the first spine aperture and the first electrode aperture are aligned along the length of the spine; and a second pin disposed within the second spine aperture and the second electrode aperture when the second spine aperture and the second electrode aperture are aligned along the length of the spine.
Clause 14. The assembly of Clause 13, the first pin and the second pin comprise a non-conductive material.
Clause 15. The assembly of Clause 13 or 14, the first electrode aperture and the second electrode aperture comprising non-conductive internal surfaces.
Clause 16. The assembly of any of Clauses 13-15, a first pin head of the first pin comprising a beveled profile, and a second pin head of the second pin comprising a beveled profile.
Clause 17. The assembly of any of Clauses 13-16, the first pin and the second pin include rivets that are compressible to form a beveled end.
Clause 18. The assembly of Clause 13 further comprising a first tab and a second tab, the first tab comprising the first electrode aperture, and the second tab comprising the second electrode aperture.
Clause 19. The assembly of Clause 18, the first tab and the second tab positioned at opposite ends of the first electrode in a direction along the length of the spine.
Clause 20. The assembly of Clause 18 or 19, the first electrode aperture and the second electrode aperture comprising non-conductive internal surfaces.
Clause 21. The assembly of any of Clauses 18-20, the first tab and the second tab being attached to and extending from the first electrode.
Clause 22. An expandable basket assembly comprising: at least one spine extending along a longitudinal axis, the spine comprising a first spine aperture along a length of the spine; a first electrode slidable along the length of the spine, the first electrode comprising a first electrode aperture; and a first rivet disposed within the first spine aperture and the first electrode aperture when the first spine aperture and the first electrode aperture are aligned along the length of the spine.
Clause 23. The assembly of Clause 22, the first rivet comprises a non-conductive material.
Clause 24. The assembly of Clause 22 or 23, the first electrode aperture comprises a non-conductive internal surface.
Clause 25. The assembly of any of Clauses 22-24, a rivet head of the first rivet comprising a beveled profile.
Clause 26. The assembly of any of Clauses 22-25, the rivet can comprise a multi-piece component comprising a rivet top connectable to a rivet bottom.
Clause 27. The assembly of Clause 26, the rivet top and the rivet bottom being positioned at opposing sides of the first electrode when connected.
Clause 28. The assembly of any of Clauses 22-27 further comprising: a second electrode slidable along the length of the spine, the second electrode comprising a second electrode aperture; and a second rivet, the spine comprising a second spine aperture along the length of the spine, and the second rivet is disposed within the second spine aperture and the second electrode aperture when the second spine aperture and the second electrode aperture are aligned along the length of the spine.
Clause 29. The assembly of any of Clauses 22-27 further comprising a second rivet, the spine comprising a second spine aperture, the first electrode comprising a second electrode aperture, and the second rivet being disposed within the second spine aperture and the second electrode aperture when the second spine aperture and the second electrode aperture are aligned along the length of the spine.
Clause 30. The assembly of Clause 29 further comprising a first tab and a second tab, the first tab comprising the first electrode aperture, and the second tab comprising the second electrode aperture.
Clause 31. The assembly of Clause 30, the first tab and the second tab positioned at opposite ends of the first electrode in a direction along the length of the spine.
Clause 32. The assembly of any of Clauses 30-31, the first tab and the second tab being attached to and extending from the first electrode.
Clause 33. The assembly of any of Clauses 27-29, the second electrode aperture comprises a non-conductive internal surface.
Clause 34. An expandable basket assembly comprising: at least one spine extending along a longitudinal axis, the spine comprising a first spine attachment along a length of the spine; and a first electrode slidable along the length of the spine, the first electrode comprising an extension insertable into the first spine attachment and compressible to form a compressed base.
Clause 35. The assembly of Clause 34, the first spine attachment is an aperture along the length of the spine.
Clause 36. The assembly of Clause 34, the at least one spine comprises a slot along at least a portion of the length of the spine, and the first spine attachment is a position along the length of the spine where the first electrode is secured to the at least one spine.
Clause 37. The assembly of any of Clauses 34-36, the extension comprises a non-conductive material.
Clause 38. A method of attaching an electrode to an elongated member, the method comprising: placing a first electrode at an end of the elongated member; sliding the first electrode along a length of the member until a first electrode aperture of the first electrode aligns with a first aperture of the member; and inserting a first pin into the first electrode aperture and the first aperture to affix the first electrode to the member.
Clause 39. The method of Clause 38, placing the first electrode at the end of the member comprises placing the end of the member into a slot of the first electrode, the member being slidable through the slot.
Clause 40. The method of Clause 38 or 39 further comprising inserting a second pin into a second electrode aperture and a second aperture to affix the first electrode to the member.
Clause 41. The method of Clause 40, the first electrode aperture is disposed within a first tab, and the second electrode aperture is disposed within a second tab, the first tab and the second tab positioned on opposite sides from a main body of the first electrode.
Clause 42. The method of any of Clauses 38-41, the first pin can comprise a rivet having a first rivet head, and the method further comprises deforming a bottom of the first pin to secure the first pin within the first electrode aperture and the first aperture.
Clause 43. The method of any of Clauses 38-41, the first pin can comprise a rivet having a first rivet head, and the method further comprises connecting a rivet bottom to the first pin to secure the first pin within the first electrode aperture and the first aperture.
Clause 44. The method of any of Clauses 38-43, sliding the first electrode along a length of the member until a first electrode aperture of the first electrode aligns with a first aperture of the member comprises sliding the first electrode past a second aperture, and the method further comprises: placing a second electrode at the end of the member; sliding the second electrode along the length of the member until a second electrode aperture of the second electrode aligns with the second aperture; and inserting a second pin into the first electrode aperture and the second aperture to affix the second electrode to the member.
Clause 45. A method of attaching an electrode to a spine of a basket catheter, the method comprising: sliding a first electrode along a length of the spine until: a first electrode aperture of the first electrode aligns with a first spine aperture of the spine; and a second electrode aperture of the first electrode aligns with a second spine aperture of the spine; inserting a first pin into the first electrode aperture and the first spine aperture to affix the first electrode to the spine; and inserting a second pin into the second electrode aperture and the second spine aperture.
Clause 46. The method of Clause 45 further comprising placing the end of the spine into a spine slot of the first electrode, the spine being slidable through the spine slot.
Clause 47. The method of Clause 45 or 46, the first electrode aperture is disposed within a first tab, and the second electrode aperture is disposed within a second tab, the first tab and the second tab extending opposite ends of a main body of the first electrode.
Clause 48. The method of any of Clauses 45-47, the first pin and the second pin are rivets having a rivet head, and the method further comprises deforming bottoms of the first pin and the second pin to secure the pins within their respective apertures.
Clause 49. The method of any of Clauses 45-48, the first pin can comprise a rivet having a first rivet head, the second pin can comprise a rivet having a second rivet head, and the method further comprises: connecting a first rivet bottom to the first pin to secure the first pin within the first electrode aperture and the first spine aperture; and connecting a second rivet bottom to the second pin to secure the second pin within the second electrode aperture and the second spine aperture.
Clause 50. The method of any of Clauses 45-49, sliding the first electrode along a length of the spine until a first electrode aperture of the first electrode aligns with a first spine aperture of the spine comprises sliding the first electrode past a third spine aperture and a fourth spine aperture, and the method further comprises: sliding a second electrode along the length of the spine until: a third electrode aperture of the second electrode aligns with a third spine aperture of the spine; and a fourth electrode aperture of the second electrode aligns with a fourth spine aperture of the spine; inserting a third pin into the third electrode aperture and the third spine aperture to affix the second electrode to the spine; and inserting a fourth pin into the fourth electrode aperture and the fourth spine aperture.
Clause 51. A method of securing an electrode to an elongated member, the method comprising: sliding a first tab along a length of a spine until a first electrode aperture of the first tab aligns with a first spine aperture of the spine; inserting a first pin into the first electrode aperture and the first spine aperture to affix the first tab to the spine; sliding a first electrode along the length of the spine until the first electrode contacts the first tab; sliding a second tab along the length of the spine until a second electrode aperture of the second tab aligns with a second spine aperture of the spine; and inserting a second pin into the second electrode aperture and the second spine aperture to affix the second tab to the spine.
Clause 52. The method of Clause 51 further comprising placing an end of the spine into a spine slot of the first electrode, the spine being slidable through the spine slot.
Clause 53. The method of Clause 51 or 52, the first pin and the second pin are rivets having a rivet head, and the method further comprises deforming bottoms of the first pin and the second pin to secure the pins within the respective electrode aperture.
Clause 54. A method of attaching an electrode to a spine of a basket catheter, the method comprising: sliding a first electrode along a length of the spine until a first electrode aperture of the first electrode aligns with a first spine aperture of the spine; inserting a first rivet into the first electrode aperture and the first spine aperture; and riveting the first rivet to affix the first electrode to the spine.
Clause 55. The method of Clause 54 further comprising placing the end of the spine into a spine slot of the first electrode, the spine being slidable through the spine slot.
Clause 56. The method of Clause 54 or 55, riveting the first rivet comprises connecting a rivet bottom to the first rivet to affix the first electrode to the spine.
Clause 57. The method of any of Clauses 54-56, sliding the first electrode along a length of the spine until a first electrode aperture of the first electrode aligns with a first spine aperture of the spine comprises sliding the first electrode past a second spine aperture, and the method further comprises: sliding a second electrode along a length of the spine until a second electrode aperture of the second electrode aligns with the second spine aperture; inserting a second rivet into the second electrode aperture and the second spine aperture; and riveting the second rivet to affix the second electrode to the spine.
Clause 58. A method of securing an electrode to an elongated member, the method comprising: sliding a first electrode along a length of a spine until it reaches a spine attachment on the spine; and compressing an extension of the first electrode to form a compressed base at an end of the extension, thereby securing the first electrode to the spine at a location of the spine attachment.
Clause 59. The method of Clause 58, the spine attachment is an aperture within the spine.
Clause 60. The method of Clause 58, the spine attachment is a position along a slot running at least a portion of the length of the spine.
Clause 61. The method of Clause 60, the extension has a rectangular cross section, a first width of which corresponds to a second width of the slot.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. A spine assembly comprising:
an elongated member extending along a longitudinal axis member comprising a first aperture along a length of the member;
a first electrode slidable along the length of the member, the first electrode comprising a first electrode aperture; and
a first pin disposed within the first aperture and the first electrode aperture when the first aperture and the first electrode aperture are aligned along the length of the member.

2. The assembly of Claim 1, the first pin comprises a non-conductive material.

3. The assembly of Claim 1 or Claim 2, the first electrode aperture comprises a non-conductive internal surface.

4. The assembly of any preceding Claim, a pin head of the first pin comprising a beveled profile.

5. The assembly of any preceding Claim, the first pin comprises a rivet comprising a bottom that is compressible to form a second head opposite a first head, optionally the first head and the second head of the rivet being positioned at opposing sides of the first electrode.

6. The assembly of any preceding Claim further comprising:
a second electrode slidable along the length of the member, the second electrode comprising a second electrode aperture; and
a second pin,
the member comprising a second aperture along the length of the member, and
the second pin is disposed within the second aperture and the second electrode aperture when the second aperture and the second electrode aperture are aligned along the length of the member.

7. The assembly of any of Claims 1 to 5 further comprising a second pin, the member comprising a second aperture, the first electrode comprising a second electrode aperture, and the second pin being disposed within the second aperture and the second electrode aperture when the second aperture and the second electrode aperture are aligned along the length of the member.

8. The assembly of Claim 7 further comprising a first tab and a second tab, the first tab comprising the first electrode aperture, and the second tab comprising the second electrode aperture, optionally the first tab and the second tab positioned at opposite ends of the first electrode in a direction along the length of the member.

9. The assembly of Claim 7 or Claim 8, wherein the second electrode aperture comprises a non-conductive internal surface.

10. The assembly of any of Claims 7 to 9, the first tab and the second tab being attached to and extending from the first electrode.

11. An expandable basket assembly comprising:
at least one spine extending along a longitudinal axis and configured to bow radially outward from the longitudinal axis, the spine comprising a first spine aperture and a second spine aperture disposed along a length of the spine;
a first electrode slidable along the length of the spine, the first electrode comprising a first electrode aperture and a second electrode aperture;
a first pin disposed within the first spine aperture and the first electrode aperture when the first spine aperture and the first electrode aperture are aligned along the length of the spine; and
a second pin disposed within the second spine aperture and the second electrode aperture when the second spine aperture and the second electrode aperture are aligned along the length of the spine.

12. The assembly of Claim 11, wherein the first pin and the second pin comprise a non-conductive material and/or the first electrode aperture and the second electrode aperture comprise non-conductive internal surfaces.

13. The assembly of Claim 11 further comprising a first tab and a second tab, the first tab comprising the first electrode aperture, and the second tab comprising the second electrode aperture, optionally the first tab and the second tab positioned at opposite ends of the first electrode in a direction along the length of the spine.

14. An expandable basket assembly comprising:
at least one spine extending along a longitudinal axis, the spine comprising a first spine aperture along a length of the spine;
a first electrode slidable along the length of the spine, the first electrode comprising a first electrode aperture; and
a first rivet disposed within the first spine aperture and the first electrode aperture when the first spine aperture and the first electrode aperture are aligned along the length of the spine.

15. The assembly of Claim 14, the first rivet comprises a non-conductive material and/or the first electrode aperture comprises a non-conductive internal surface.
